# EUROPEAN PATENT APPLICATION

(11) **EP 4 364 636 A1**
(43) Date of publication of application: **08.05.2024**
(21) Application number: 21948283.3
(22) Date of filing: 29.06.2021
(51) Int. Cl.: A61B 1/045

(54) **IMAGE PROCESSING DEVICE, IMAGE PROCESSING METHOD, AND STORAGE MEDIUM**

(71) Applicant: NEC Corporation, 108-8001 Tokyo (JP)
(72) Inventor: SHINO, Ryosaku, Tokyo 108-8001 (JP)
(74) Representative: Betten & Resch
(86) International application number: PCT/JP2021/024505
(87) International publication number: WO 2023/275974

(57) **Abstract**

The image processing device 1X mainly includes a three-dimensional reconstruction means 31X, a matching means 32X, and a display control means 33X. The three-dimensional reconstruction means 31X generates reconstructed data obtained by three-dimensionally reconstructing an examination target, based on endoscopic images of the examination target captured by a photographing unit provided in an endoscope. The matching means 32X performs matching between a three-dimensional model of the examination target and the reconstructed data. The display control means 33X displays information regarding meta data associated with the three-dimensional model, based on a result of the matching.

## Description

### TECHNICAL FIELD

The present disclosure relates to a technical field of an image processing device, an image processing method, and a storage medium for processing an image acquired in endoscopic examination.

### BACKGROUND

An endoscopic examination system for displaying images taken in the lumen of an organ is known. For example, Patent Literature 1 discloses a technique for generating, on the basis of images taken by endoscope, three-dimensional model data of the examination target to thereby display a three-dimensional model image. Patent Literature 2 discloses a technique for generating volume data (volumetric image) representing a large bowel by capturing a three-dimensional region in which the large bowel is included by an X-ray CT device. In addition, Non-Patent Literature 1 discloses a technique for reconstructing the three-dimensional shape of a stomach from captured images using the SfM (Structure from Motion) method. Furthermore, Non-Patent Literature 2 discloses a non-rigid alignment method of three-dimensional shapes.

### CITATION LIST

### PATENT LITERATURE

Patent Literature 1: WO2017/203814
Patent Literature 2: JP2011-139797A

### NON-PATENT LITERATURE

Non-Patent Literature 1: Aji Resindra Widya et al. "3D Reconstruction of Whole Stomach from Endoscope Video Using Structure-from-Motion", 2019 41st Annual International Conference of the IEEE Engineering in Medicine and Biology Society, pp. 3900-3904.
Non-Patent Literature 2: Dai, H et al. "Non-rigid 3D Shape Registration using an Adaptive Template", The European Conference on Computer Vision (ECCV) Workshops, 2018.

### SUMMARY

### PROBLEM TO BE SOLVED

In some cases, a preliminary examination is performed prior to endoscopic examination using a CT device or an MRI device. In these cases, it is preferable that the information specified in the preliminary examination can be utilized also in the endoscopic examination.

In view of the above-described issue, it is therefore an example object of the present disclosure to provide an image processing device, an image processing method, and a storage medium capable of suitably utilizing, in an endoscope examination, information obtained in advance.

### MEANS FOR SOLVING THE PROBLEM

One mode of the image processing device is an image processing device including:
a three-dimensional reconstruction means configured to generate reconstructed data obtained by three-dimensionally reconstructing an examination target, based on endoscopic images of the examination target captured by a photographing unit provided in an endoscope;
a matching means configured to perform matching between a three-dimensional model of the examination target and the reconstructed data; and
a display control means configured to display information regarding meta data associated with the three-dimensional model, based on a result of the matching.

One mode of the image processing method is an image processing method executed by a computer, the image processing method including:
generating reconstructed data obtained by three-dimensionally reconstructing an examination target, based on endoscopic images of the examination target captured by a photographing unit provided in an endoscope;
performing matching between a three-dimensional model of the examination target and the reconstructed data; and
displaying information regarding meta data associated with the three-dimensional model, based on a result of the matching.

One mode of the storage medium is a storage medium storing a program executed by a computer, the program causing the computer to:
generate reconstructed data obtained by three-dimensionally reconstructing an examination target, based on endoscopic images of the examination target captured by a photographing unit provided in an endoscope;
perform matching between a three-dimensional model of the examination target and the reconstructed data; and
display information regarding meta data associated with the three-dimensional model, based on a result of the matching.

### EFFECT

An example advantage according to the present invention is to suitably utilizing, in an endoscope examination, information obtained in advance.

### BRIEF DESCRIPTION OF THE DRAWINGS

[FIG. 1] It illustrates a schematic configuration of an endoscopic examination system.
[FIG. 2] It is a hardware configuration of an image processing device.
[FIG. 3] It is a functional block diagram of the image processing device.
[FIG. 4] It is a diagram showing an outline of the processing in the three-dimensional reconstruction unit and the matching unit.
[FIG. 5] It illustrates an example of a flowchart showing an outline of a display process performed by the image processing device during the endoscopic examination in the first example embodiment.
[FIG. 6] It illustrates a first display example of the examiner confirmation screen image.
[FIG. 7] It illustrates a second display example of the examiner confirmation screen image.
[FIG. 8] It illustrates a third display example of the examiner confirmation screen image.
[FIG. 9] It illustrates a fourth display example of the examiner confirmation screen image.
[FIG. 10] It illustrates a fifth display example of the examiner confirmation screen image.
[FIG. 11] It illustrates a sixth display example of the examiner confirmation screen image.
[FIG. 12] It is a block diagram of an image processing device according to a second example embodiment.
[FIG. 13] It illustrates an example of a flowchart showing a processing procedure executed by the image processing device according to the second example embodiment.

### EXAMPLE EMBODIMENTS

Hereinafter, example embodiments of an image processing device, an image processing method, and a storage medium will be described with reference to the drawings.

### <First Example Embodiment>

### (1) System Configuration

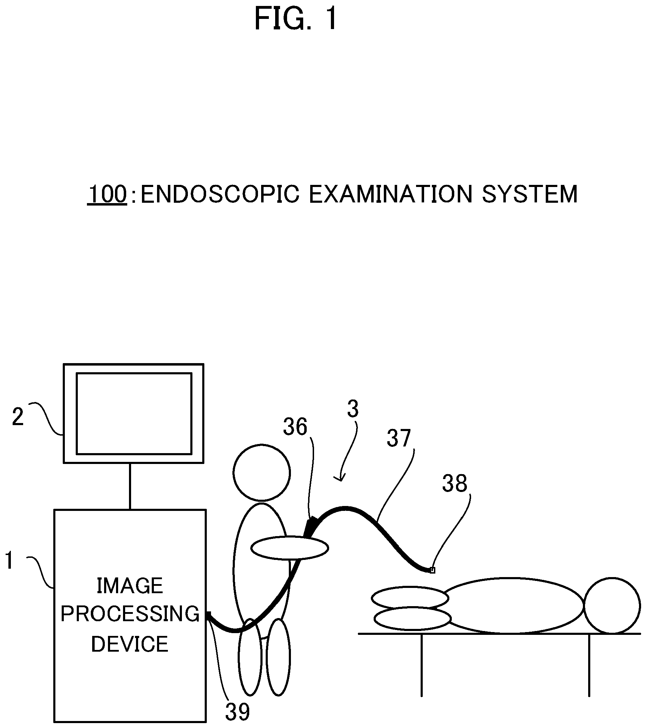

FIG. 1 shows a schematic configuration of an endoscopic examination system 100. In an endoscopic examination (including any treatment), the endoscopic system 100 presents, together with an endoscopic image obtained in real time, information relating to a part (lesion part) which is suspected of lesion and which was detected based on a preliminary examination conducted prior to the endoscopic examination. Thereby, the endoscopic examination system 100 assists an examiner such as a doctor in charge of the endoscopic examination. It is noted that the above-mentioned preliminary examination is an examination in which scan data of an organ to be an examination target is generated by a CT device or an MRI device or the like and a diagnosis is made based on the generated scan data. The diagnosis described above may be made by a doctor or by a computer.

As shown in FIG. 1, the endoscopic examination system 100 mainly includes an image processing device 1, a display device 2, and an endoscope 3 connected to the image processing device 1.

The image processing device 1 acquires an image (also referred to as "endoscopic image Ic") captured by the endoscope 3 in time series from the endoscope 3 and displays a screen image (also referred to as "examiner confirmation screen image") for confirmation by an examiner of the endoscopic examination on the display device 2. The endoscopic image Ic is an image captured at predetermined time intervals in at least one of the insertion process of the endoscope 3 to a subject or the ejection process of the endoscope 3 from the subject. In the present example embodiment, the image processing device 1 generates data (also referred to as " reconstructed data Mr") obtained by reconstructing a three-dimensional shape of an organ (digester) that is the examination target of the subject from endoscopic images Ic, and then matches the reconstructed data Mr with a three dimensional model (also referred to as "preliminary examination model Mp") of the organ that is the examination target of the subject, wherein the preliminary examination model Mp is generated based on results of the preliminary examination using a CT device or an MRI device. Then, on the basis of the matching result, the image processing device 1 displays on the display device 2 the examiner confirmation screen image in which the position of the lesion part or the like detected in the preliminary examination is highlighted on the endoscopic image Ic.

The display device 2 is a display or the like for displaying information based on a display signal supplied from the image processing device 1.

The endoscope 3 mainly includes an operation unit 36 for examiner to perform a predetermined input, a shaft 37 which has flexibility and which is inserted into the organ to be photographed of the subject, a tip unit 38 having a built-in photographing unit such as an ultrasmall image pickup device, and a connecting unit 39 for connecting with the image processing device 1.

In the following description, an explanation will be given on the assumption that a stomach is mainly targeted in the endoscopic examination, however examples of the examination target include not only the stomach but also any other digestive tract (digestive organ) such as a large bowel, an esophageal, a small bowel and a duodenum. Examples of the endoscope in the present disclosure include a laryngendoscope, a bronchoscope, an upper digestive tube endoscope, a duodenum endoscope, a small bowel endoscope, a large bowel endoscope, a capsule endoscope, a thoracoscope, a laparoscope, a cystoscope, a cholangioscope, an arthroscope, a spinal endoscope, a blood vessel endoscope, and an epidural endoscope.

The target part of detection in the preliminary examination and the endoscopic examination is not limited to a lesion part, and it may be any point (also referred to as "attention part") which needs an attention of the examiner. Examples of such an attention part include a lesion part, an inflammation part, a point with an operating mark or other cuts, a point with a fold or a protrusion, a point on the wall surface of the lumen where the tip unit 38 of the endoscope 3 tends to get contact (caught). When the attention part is the lesion part, the conditions of the lesion part to be detected in endoscopic examination are exemplified as (a) to (f) below.
(a) Head and neck: pharyngeal cancer, malignant lymphoma, papilloma
(b) Esophagus: esophageal cancer, esophagitis, esophageal hiatal hernia, Barrett's esophagus, esophageal varices, esophageal achalasia, esophageal submucosal tumor, esophageal benign tumor
(c) Stomach: gastric cancer, gastritis, gastric ulcer, gastric polyp, gastric tumor
(d) Duodenum: duodenal cancer, duodenal ulcer, duodenitis, duodenal tumor, duodenal lymphoma
(e) Small bowel: small bowel cancer, small bowel neoplastic disease, small bowel inflammatory disease, small bowel vascular disease
(f) Large bowel: colorectal cancer, colorectal neoplastic disease, colorectal inflammatory disease; colorectal polyps, colorectal polyposis, Crohn's disease, colitis, intestinal tuberculosis, hemorrhoids.

### (2) Hardware Configuration

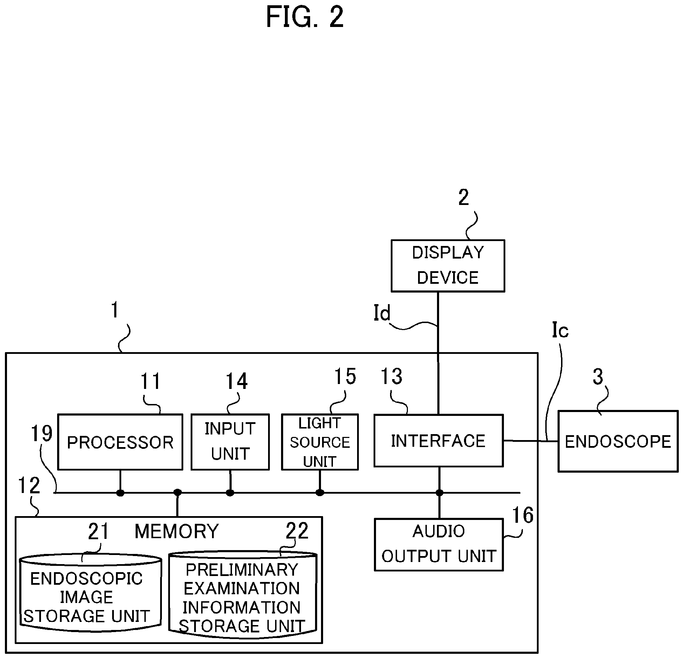

FIG. 2 shows the hardware configuration of the image processing device 1. The image processing device 1 mainly includes a processor 11, a memory 12, an interface 13, an input unit 14, a light source unit 15, and an audio output unit 16. Each of these elements is connected via a data bus 19.

The processor 11 executes a predetermined process by executing a program or the like stored in the memory 12. The processor 11 is one or more processors such as a CPU (Central Processing Unit), a GPU (Graphics Processing Unit), and a TPU (Tensor Processing Unit). The processor 11 may be configured by plural processors. The processor 11 is an example of a computer.

The memory 12 is configured by a variety of volatile memories which is used as working memories, and nonvolatile memories which stores information necessary for the process to be executed by the image processing device 1, such as a RAM (Random Access Memory) and a ROM (Read Only Memory). The memory 12 may include an external storage device such as a hard disk connected to or built in to the image processing device 1, or may include a storage medium such as a removable flash memory. The memory 12 stores a program for the image processing device 1 to execute each process in the present example embodiment.

The memory 12 functionally includes an endoscopic image storage unit 21 and a preliminary examination information storage unit 22.

The endoscopic image storage unit 21 stores a series of endoscopic images Ic taken by the endoscope 3 in the endoscopic examination based under the control of the processor 11. These endoscopic images Ic are images used for generating the reconstructed data Mr. For example, the endoscopic images Ic are stored in the endoscopic image storage unit 21 in association with the identification information (e.g., patient ID) of the subject and time stamp information.

The preliminary examination information storage unit 22 stores the preliminary examination information that is information regarding the examination result of the preliminary examination of the subject using a CT device or an MRI device or the like. The preliminary examination information includes: scan data (also referred to as "preliminary scan data") of an organ of the subject subjected to the examination such as a CT device or an MRI device; a preliminary examination model Mp that is a three-dimensional shape model of the target organ of the examination generated from the preliminary scan data; and meta data associated with the preliminary scan data and the preliminary examination model Mp.

For example, the above-mentioned meta data is data which is attached to the preliminary scan data through an annotation work by a doctor in charge of the preliminary examination, or, data obtained by applying a CAD (Computer Aided Diagnosis) to the preliminary scan data. For example, the above-described annotation work is a work to be conducted by a doctor in charge of the preliminary examination as to specifying an attention part of preliminary scan data with reference to the displayed preliminary scan data and inputting a comment or the like regarding the specified attention part of preliminary scan data to a computer. Then, the meta data includes information regarding the attention part such as a lesion part detected in the preliminary examination. For example, the meta data includes position information (e.g., a coordinate value in a coordinate system used in the preliminary scan data) which specifies an attention part to be noticed in the endoscopic examination and content information representing a diagnosis result or the like regarding the position (that is, the attention part) specified by the position information. The meta data may also include information regarding the attributes of one or more doctors in charge of the preliminary examination (including the name of the doctor in charge and the affiliation information thereof), as will be described later.

The preliminary examination model Mp is generated by extracting a three-dimensional shape of the target organ of the examination from preliminary scan data such as three-dimensional CT images and MRI data. For example, the preliminary examination model Mp is herein represented in a predetermined three-dimensional coordinate system. The preliminary examination information storage unit 22 may further include coordinate transformation information between the three-dimensional coordinate system of the preliminary examination model Mp and the coordinate system (two-dimensional or three-dimensional coordinate system) of the preliminary scan data. This coordinate transformation information is generated in the process of generating a preliminary examination model Mp from the preliminary scan data. The process of generating the preliminary examination model Mp from the preliminary scan data may be performed in advance by the image processing device 1 before the endoscopic examination, or may be performed by a device other than the image processing device 1 before the endoscopic examination.

Here, at least either the endoscopic image storage unit 21 or the preliminary examination information storage unit 22 may be provided in, instead of the memory 12, an external device capable of wired or wireless data communication with the image processing device 1. In this case, the external device may be one or more server devices capable of data communication with the image processing device 1 via a communication network.

In addition to the above-described information, the memory 12 may store various kinds of information necessary for processing in the present example embodiment. For example, when the image processing device 1 performs a CAD based on the endoscopic images Ic, the memories 12 may further store parameters and the like regarding a lesion detection model required to perform the CAD. In this case, the lesion detection model is, for example, a machine learning model such as a neural network and a support vector machine, and is configured to output, when an endoscopic image Ic is inputted thereto, the presence or absence of a lesion part in the inputted endoscopic image Ic and, if there is a lesion part, its position information (which may be region information) in the inputted endoscopic image Ic. In the case where the lesion detection model is configured by a neural network, the memory 12 stores various parameters such as, for example, a layer structure, a neuron structure of each layer, the number of filters and the size of filters in each layer, and the weight for each element of each filter.

The interface 13 performs an interface operation between the image processing device 1 and an external device. For example, the interface 13 supplies the display information "Id" generated by the processor 11 to the display device 2. Further, the interface 13 supplies the light generated by the light source unit 15 to the endoscope 3. The interface 13 also provides an electrical signal to the processor 11 indicative of the endoscopic image Ic supplied from the endoscope 3. The interface 13 may be a communication interface, such as a network adapter, for wired or wireless communication with the external device, or a hardware interface compliant with a USB (Universal Serial Bus), a SATA (Serial AT Attachment), or the like.

The input unit 14 generates an input signal based on the operation by the examiner. Examples of the input unit 14 include a button, a touch panel, a remote controller, and a voice input device. The light source unit 15 generates light for supplying to the tip unit 38 of the endoscope 3. The light source unit 15 may also incorporate a pump or the like for delivering water and air to be supplied to the endoscope 3. The audio output unit 16 outputs a sound under the control of the processor 11.

### (3) Functional Block

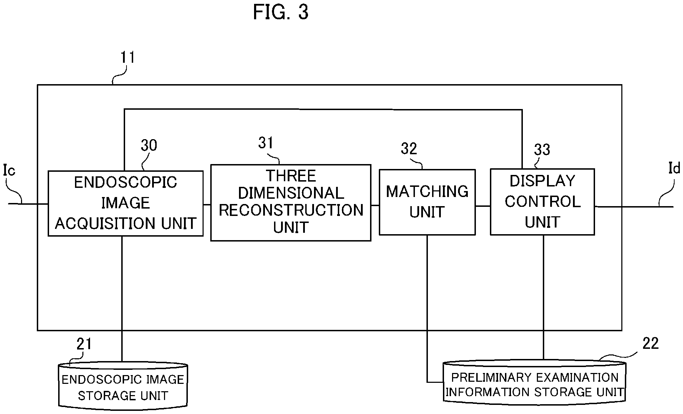

FIG. 3 is a functional block diagram of the image processing device 1. As shown in FIG. 3, the processor 11 of the image processing device 1 functionally includes an endoscope image acquisition unit 30, a three-dimensional reconstruction unit 31, a matching unit 32, and a display control unit 33. In FIG. 3, blocks to exchange data with each other are connected by a solid line, but the combination of the blocks to exchange data is not limited to FIG. 3. The same applies to the drawings of other functional blocks described below.

The endoscopic image acquisition unit 30 acquires an endoscopic image Ic taken by the endoscope 3 through the interface 13 at predetermined intervals. Then, the endoscopic image acquisition unit 30 supplies the acquired endoscopic image Ic to the three-dimensional reconstruction unit 31. In addition, the endoscopic image acquisition unit 30 stores the acquired endoscopic image Ic in the endoscopic image storage unit 21 in association with the time stamp, patient ID, and the like. The endoscopic image acquisition unit 30 supplies the most recently acquired endoscopic image Ic to the display control unit 33.

The three-dimensional reconstruction unit 31 generates reconstructed data Mr indicating the three-dimensional shape of the photographed organ on the basis of a plurality of endoscopic images Ic acquired by the endoscopic image acquisition unit 30 in the endoscopic examination. The reconstructed data Mr includes, for example, point cloud data of three-dimensional position information.

In this instance, for example, upon acquiring a number of endoscopic images Ic required for generating the reconstructed data Mr, the three-dimensional reconstruction unit 31 constructs the reconstructed data Mr using a technique for reconstructing the three-dimensional shape of the subject and the relative position of the photographing unit from a plurality of images. Examples of such a technique include the Structure from Motion (SfM). Thereafter, each time acquiring a predetermined number of endoscopic images Ic, the three-dimensional reconstruction unit 31 updates the reconstructed data Mr. The predetermined number may be one or more and is predetermined to a value in consideration of the treatment capacity of the image processing device 1, for example. The three-dimensional reconstruction unit 31 supplies the generated (or updated) reconstructed data Mr to the matching unit 32. The method of generating the reconstructed data Mr will be described later.

The matching unit 32 performs matching between the reconstructed data Mr supplied from the three-dimensional reconstruction unit 31 and the preliminary examination model Mp stored in the preliminary examination information storage unit 22, and supplies the matching result "Rm" which is the result of the matching to the display control unit 33. In this instance, for example, the matching unit 32 performs a non-rigid alignment therebetween and generates data representing the reconstructed data Mr and the preliminary examination model Mp subjected to the non-rigid alignment in a common three-dimensional coordinate system (also referred to as "common coordinate system"). Then, for example, the matching unit 32 generates a matching result Rm that includes the above-mentioned generated data and/or coordinate transformation information regarding the above-described common coordinate system. For example, the above-described coordinate transformation information herein includes coordinate transformation information from the coordinate system used in the reconstructed data Mr to the common coordinate system, and coordinate transformation information from the coordinate system used in the preliminary examination model Mp to the common coordinate system.

The display control unit 33 generates display information Id regarding the examiner confirmation screen image, based on the matching result Rm generated by the matching unit 32, and supplies the generated display information Id to the display device 2, thereby causing the display device 2 to display the examiner confirmation screen image. In this instance, the display control unit 33 causes the display device 2 to display, on the examiner confirmation screen image, information regarding the attention part, which is indicated by the meta data stored in the preliminary examination information storage unit 22, in association with the endoscopic image Ic supplied from the endoscopic image acquisition unit 30. The display control unit 33 may also output information for providing a guidance or a warning regarding the operation of the endoscope 3 by the examiner. The information may be outputted on the examiner confirmation screen image or may be outputted by the audio output unit 16.

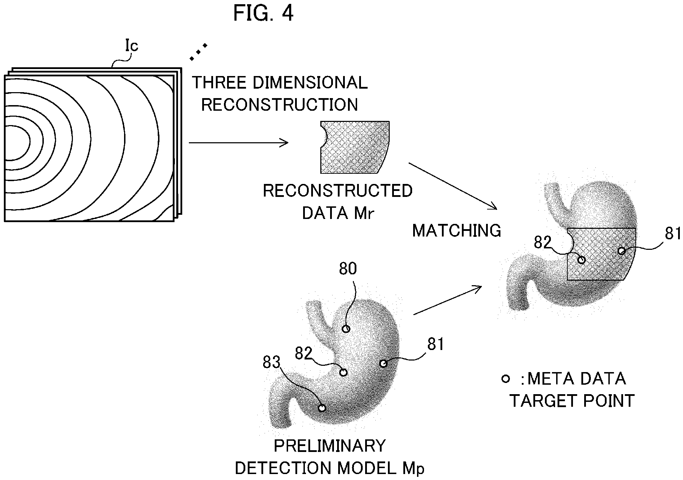

FIG. 4 is a diagram showing an outline of processing in the three-dimensional reconstruction unit 31 and the matching unit 32. For convenience of explanation, FIG. 4 shows an example in which the stomach is examined. The outline of the processing shown in FIG. 4 is similarly applied to examples of the large bowel and other digestive tracts.

On the preliminary examination model Mp shown in FIG. 4, the points (also referred to as "meta data target parts") 80 to 83 that are targets of the meta data are clearly shown. In other words, the meta data target parts 80 to 83 are locations specified by the position information included in the meta data and are mentioned in the content information of the meta data. The meta data target parts 80 to 83 can be represented in the coordinate system of the preliminary examination model Mp based on the meta data stored in the preliminary examination information storage unit 22.

The three-dimensional reconstruction unit 31 generates reconstructed data Mr corresponding to the three-dimensional shape of the region (already-photographed region) in the digestive tract already photographed by the endoscope 3, on the basis of a plurality of endoscopic images Ic acquired up to the present during the endoscopic examination.

Then, the matching unit 32 performs matching (non-rigid alignment) between the preliminary examination model Mp stored in the preliminary examination information storage unit 22 and the reconstructed data Mr. Accordingly, the matching unit 32 associates the preliminary examination model Mp representing the whole examination target with the reconstructed data Mr corresponding to the already-photographed region in the common coordinate system, and generates a matching result Rm representing the association result (e.g., coordinate transformation information from each data to data in the common coordinate system). In addition, through the coordinate transformation to the common coordinate system, the matching unit 32 identifies the positional correspondence between the reconstructed data Mr (that is, the endoscopic images Ic constituting the reconstructed data Mr) and the meta data target parts 80 to 83 represented in the coordinate system of the preliminary examination model Mp. In FIG. 4 that illustrates the state after the matching, the meta data target parts 81 and 82 superimposed on the reconstructed data Mr are explicitly shown.

Based on the matching result Rm and the meta data, the display control unit 33 generates display information Id regarding the examiner confirmation screen image including an endoscopic image Ic in which the meta data target parts are highlighted.

Each component of the the endoscope image acquisition unit 30, the three-dimensional reconstruction unit 31, the matching unit 32 and the display control unit 33 can be realized, for example, by the processor 11 which executes a program. In addition, the necessary program may be recorded in any non-volatile storage medium and installed as necessary to realize the respective components. In addition, at least a part of these components is not limited to being realized by a software program and may be realized by any combination of hardware, firmware, and software. At least some of these components may also be implemented using user-programmable integrated circuitry, such as FPGA (Field-Programmable Gate Array) and microcontrollers. In this case, the integrated circuit may be used to realize a program for configuring each of the above-described components. Further, at least a part of the components may be configured by a ASSP (Application Specific Standard Produce), ASIC (Application Specific Integrated Circuit) and/or a quantum processor (quantum computer control chip). In this way, each component may be implemented by a variety of hardware. The above is true for other example embodiments to be described later. Further, each of these components may be realized by the collaboration of a plurality of computers, for example, using cloud computing technology.

### (4) Processing Flow

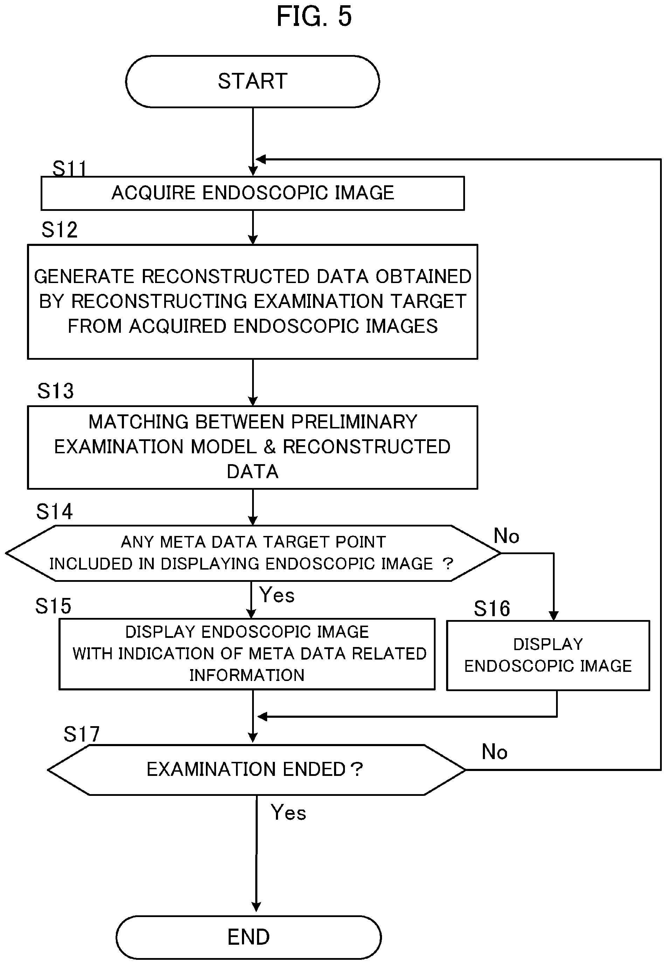

FIG. 5 is an example of a flowchart illustrating an outline of a display process that is executed by the image processing device 1 during the endoscopic examination in the first example embodiment.

First, the image processing device 1 acquires an endoscopic image Ic (step S11). In this instance, the endoscopic image acquisition unit 30 of the image processing device 1 receives the endoscopic image Ic from the endoscope 3 through the interface 13.

Next, the image processing device 1 generates reconstructed data Mr in which the examination target is three-dimensionally reconstructed from a plurality of endoscopic images Ic acquired at step S11 (step S12). In this instance, the three-dimensional reconstruction unit 31 of the image processing device 1 generates the reconstructed data Mr using a technique such as SfM on the basis of the endoscopic images Ic acquired during the period from the start of the examination to the present processing time.

Next, the image processing device 1 performs the matching between the preliminary examination model Mp and the reconstructed data Mr (step S13). In this instance, the matching unit 32 of the image processing device 1 generates the matching result Rm by performing non-rigid alignment between the preliminary examination model Mp acquired from the preliminary examination information storage unit 22 and the reconstructed data Mr generated by the three-dimensional reconstruction unit 31.

Then, based on the matching result Rm, the image processing device 1 determines whether or not there is any meta data target part in the target endoscopic image Ic of display on the display device 2 (step S14). In this instance, the endoscopic image Ic to be displayed on the display device 2 is the latest endoscopic image Ic among the endoscopic images Ic used for generating the reconstructed data Mr, for example.

Here, a supplemental description will be given of the method of determining whether or not there is a meta data target part in the target endoscopic image Ic of display on the display device 2. For example, based on the matching result Rm and the meta data, the image processing device 1 firstly recognizes meta data target parts in the common coordinate system and further converts the meta data target parts into points in the coordinate system of the target endoscopic image Ic of display on the display device 2. Then, if there is any meta data target part, among the meta data target parts after the coordinate transformation, included in the display range of the target endoscopic image Ic of display on the display device 2, the image processing device 1 determines that there is a meta data target part in the target endoscopic image Ic of display on the display device 2. Generally, when three-dimensional data is generated from a plurality of images using a technique such as SfM, coordinate transformation information between the generated three-dimensional data and the original images is obtained. Therefore, by using such coordinate transformation information, the image processing device 1 can convert a meta data target parts of the common coordinate system into a point in the coordinate system of each individual endoscopic image Ic.

Upon determining that there is a meta data target part in the target endoscopic image Ic of display on the display device 2 (step S14; Yes), the image processing device 1 causes the display device 2 to display the endoscopic image Ic with indication of the information relating to the meta data (step S15). In this instance, the display control unit 33 of the image processing device 1 generates display information Id for displaying the examiner confirmation screen image in which the endoscopic image Ic with the highlighted meta data target part and the content of the meta data target part are clearly indicated, as will be described later, and supplies the display information Id to the display device 2. On the other hand, upon determining that there is no meta data target part in the target endoscopic image Ic of display on the display device 2 (step S14; No), the image processing device 1 causes the display device 2 to display the target endoscopic image Ic (step S16).

Next, the image processing device 1 determines whether or not the endoscopic examination has ended (step S17). For example, upon detecting a predetermined input or the like to the input unit 14 or the operation unit 36, the image processing device 1 determines that the endoscopic examination has ended. Upon determining that the endoscopic examination has ended (step S17; Yes), the image processing device 1 ends the process of the flowchart. On the other hand, upon determining that the endoscopic examination has not ended (step S17; No), the image processing device 1 gets back to the process at step S11. Then, at step S11, the image processing device 1 acquires an endoscope image Ic newly generated by the endoscope scope 3, and then re-executes the processes at step S12 to step S17 additionally using the endoscope image Ic.

A supplemental description will be given of the generating process of the preliminary examination model Mp stored in the preliminary examination data storage unit 22. Hereinafter, for convenience of explanation, a description will be given of the process executed by the image processing device 1, however the process may be executed by any device other than the image processing device 1. In that case, after the preliminary examination model Mp is generated by any device, the generated preliminary examination model Mp is stored in the memory 12 (the preliminary examination information storage unit 22 in detail) through data communication or a removable storage medium or the like.

First, the image processing device 1 acquires preliminary scan data such as a 3D-CT images or MRI data obtained by photographing the target organ of the subject to be examined. Then, the image processing device 1 extracts the region of the target organ of the examination from the preliminary scan data based on the user input. In this case, for example, the image processing device 1 displays the preliminary scan data on the display device 2 and receives, from the input unit 14, a user input for specifying the region of the target organ of the examination. Then, the image processing device 1 generates volume data representing the region of the target organ of the examination extracted from the preliminary scan data regarding the subject. For example, the volume data is three-dimensional voxel data that represents the region of the target organ of the examination by using binary values that are 0 and 1. Next, from the above-described volume data, the image processing device 1 generates a three-dimensional preliminary examination model Mp that is a surface model. In this case, the image processing device 1 converts the volume data into the preliminary examination model Mp using any algorithm for converting voxel data into polygon data. Examples of the above-mentioned algorithm include the marching cube method and the marching tetrahedra method. The generated preliminary examination model Mp is stored in the memory 12 (the preliminary examination information storage unit 22 for details) which can be referred to by the image processing device 1.

Next, a description will be given of the matching process at step S13.

First, the matching unit 32 extracts feature points as landmarks from the preliminary examination model Mp and the reconstructed data Mr, respectively. In this instance, the matching unit 32 three-dimensionally smooths the reconstructed data Mr. Then, based on point cloud constituting the smoothed reconstructed data Mr and the connected graph of the point cloud, the matching unit 32 extracts the feature points that are characteristic in the point cloud. In this instance, the matching part 32 performs extraction of the above-mentioned feature points by using any of various point group feature extraction techniques, such as principal component analysis (PCA: Principal Component Analysis) and DoCoG (Difference of Center of Gravity) regarding a point cloud, for example. In the preliminary examination model Mp, a predetermined identification label or the like may be provided for each feature point to be extracted.

Next, the matching unit 32 matches (associates) the feature points extracted from the preliminary examination model Mp with the feature points extracted from the reconstructed data Mr, and performs a rigid matching (registration) between the preliminary examination model Mp and the reconstructed data Mr. In this case, the matching unit 32 translates (includes rotating) at least one of the preliminary examination model Mp and/or the reconstructed data Mr so that the distance between associated feature points is minimized. Next, the matching unit 32 morphs the preliminary examination model Mp with reference to the reconstructed data Mr. In this instance, the matching unit 32 matches the preliminary examination model Mp with the reconstructed data Mr by using a matching method between point clouds such as ICPD (Iterative Coherent Point Drift) to thereby move points included in the preliminary examination model Mp other than the points that are regarded as the feature points (landmarks).

### (5) Examiner Confirmation Screen Image

Next, a detailed description will be given of each display example (first display example to sixth display example) of the examiner confirmation screen image which the display control unit 33 displays on the display device 2. The display control unit 33 generates display information Id for displaying an examiner confirmation screen image including an endoscopic image Ic with clear indication of a meta data target part and supplies the display information Id to the display device 2, thereby causing the display device 2 to display the examiner confirmation screen image.

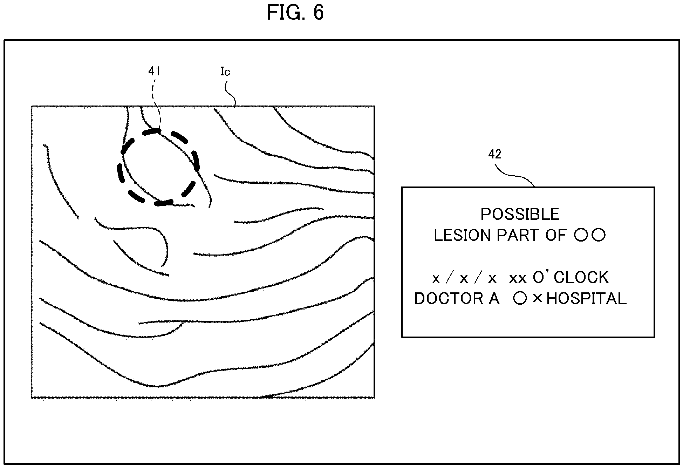

FIG. 6 shows a first display example of the examiner confirmation screen image. In the first display example, in such a case where there is a meta data target part corresponding to the endoscopic image Ic most-recently generated by the endoscope 3, the display control unit 33 displays information such as a mark for highlighting the meta data target part and a diagnosis result associated with the meta data target part.

In FIG. 6, the display control unit 33 mainly displays, on the examiner confirmation screen image, the endoscopic image Ic most-recently taken by the endoscope 3, a mark 41 that is a broken line circle circling a meta data target part in the endoscope image Ic, and a meta data display field 42 indicating the content of the meta data relating to the meta data target part highlighted by the mark 41.

Here, regarding the meta data target part indicated by the mark 41, the display control unit 33 displays, in the meta data display field 42, the diagnosis result (a comment "POSSIBLE LESION PART OF OO" in this case) in the preliminary examination and diagnostic date and time and doctor's attributes (in this case the doctor's name "A" and the hospital name "OX HOSPITAL"). In this case, the preliminary examination information storage unit 22 stores the meta data that is information regarding the above-described diagnosis result, diagnosis date and time, and the doctor's attributes in association with the position information (e.g., coordinate information represented in the coordinate system of the preliminary scan data or the like) indicating the meta data target part positioned at the center of the mark 41.

Thus, according to the first display example, the display control unit 33 can display, in association with the endoscopic image Ic captured in the endoscopic examination, the information relating to the meta data generated in the preliminary examination. Thus, in the endoscopic examination, the examiner of the endoscopic examination can accurately grasp the lesion part and other attention parts detected in the preliminary examination. Further, in the first display example, the examiner of the endoscopic examination can suitably grasp the attributes of the doctor who performed the preliminary examination on the examiner confirmation screen image.

Instead of highlighting a meta data target part by the mark 41 that is a broken line circle surrounding the meta data target part, the display control unit 33 may highlight the meta data target part on the endoscopic image Ic by the mark having any shape (e.g., star mark or any other figure) other than the broken line circle. In addition, when the meta data target part is recorded in the meta data as a region, the display control unit 33 may display the region while emphasizing the entire region or the borderline of the region on the endoscopic image Ic.

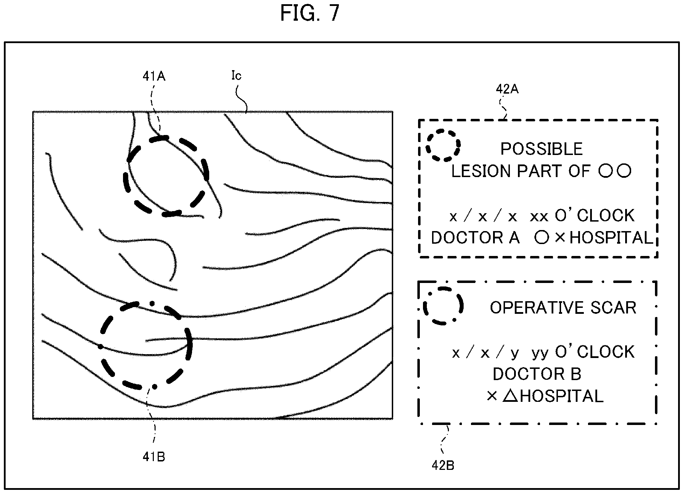

FIG. 7 shows a second display example of the examiner confirmation screen image. In the second display example, when there are plural doctors who performed the preliminary examination, the display control unit 33 differentiates the display mode relating to the meta data for each doctor who performed the preliminary examination from each other.

In FIG. 7, there are meta data target parts based on the meta data generated through annotation works by the doctor A and the doctor B in the imaging area of the endoscopic image Ic most-recently captured by the endoscope 3. Here, the display control unit 33 displays, on the most-recently acquired endoscopic image Ic, the mark 41A that is a broken line circle circling the meta data target part based on the meta data attached by the doctor A in the preliminary examination, and the mark 41B that is a dash-dotted line circle circling the meta data target part based on the meta data attached by the doctor B in the preliminary examination. Further, the display control unit 33 displays, on the examiner confirmation screen image, a meta data display field 42A indicating the content of the meta data corresponding to the meta data target part highlighted by the mark 41A and a meta data display field 42B indicating the content of the meta data corresponding to the meta data target part highlighted by the mark 41B. Here, the meta data display field 42A displays the diagnosis result (a comment indicating "POSSIBLE LESION PART OF OO", in this case) of the target meta data target part, the diagnosis date and time, and the attributes of the doctor A who made the diagnosis, and the meta data display field 42B displays the diagnosis result (a comment indicating "OPERATIVE SCAR" in this case) of the target meta data target part, the diagnosis date and time, and the attributes of the doctor B who made the diagnosis.

As described above, the display control unit 33 shows, by using dashed lines, the mark 41A and the meta data display field 42A which are display objects based on the meta data attached by the doctor A while showing, by using dash-dotted lines, the mark 41B and the meta data display field 42B which are display objects based on the meta data attached by the doctor B. In this way, in the second display example, the display control unit 33 differentiates the line type of the mark circling the meta data target part for each doctor who conducted the preliminary examination from each other. In this case, any line type such as a dashed line, a dash-dotted line, and a solid line may be used.

Thus, in the second display example, the display control unit 33 differentiates the display mode of the information (display object) relating to the meta data for each doctor who conducted the preliminary examination from each other. Thus, the display control unit 33 can make the examiner of the endoscopic examination clearly recognize the respective doctors in charge of the preliminary examination who attached the meta data. It is noted that, instead of or in addition to differentiating the line type of the mark circling the meta data target part from each other with respect to each doctor who conducted the preliminary examination, the display control unit 33 may differentiates the shape of the mark circling the meta data target part from each other with respective to each doctor who conducted the preliminary examination. Examples of the shape of the mark in this case include a round, a triangular, a square and any other shapes. The display mode of the meta data which is differentiated from each other with respective to each doctor who conducted the preliminary examination are not limited to the above-mentioned example. Any display mode for the examiner to recognize and distinguish respective doctors in charge of the preliminary examination corresponding to the meta data target parts may be used. This is true for cases of differentiating the display mode of information superimposed on the endoscopic image from each other according to any display example (e.g., the fourth display example and the sixth display example to be described later) other than the second display example. Thus, it is possible to differentiate the display mode of the information to be recognized individually by the examiner from each other, thereby to support the examiner to perform the examination.

In some embodiments, the display control unit 33 may set a display mode in which a mark (the marks 41A and 41B in FIG. 7) representing a meta data target part is not overlapped with the target meta data target part. In another example, the display control unit 33 may increase the degree of transparency of the overlapping display portion between the mark indicative of the meta data target part and the display of the meta data target part (specifically, increase the degree of transparency so that the meta data target part overlapped with the mark can be visually recognized). For example, in this case, the display control unit 33 makes the inside of the mark indicating the meta data target part transparent (i.e., makes the mark open). Thus, the display control unit 33 can assist the examiner to visually recognize the meta data target part.

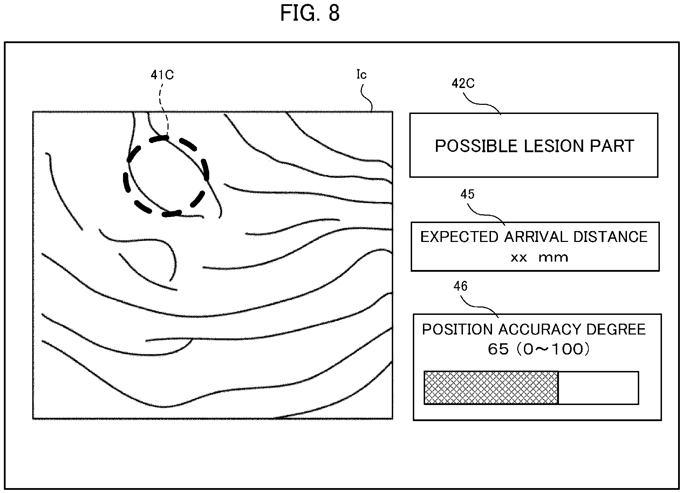

FIG. 8 shows a third display example of the examiner confirmation screen image. In the third display example, the display control unit 33 displays a degree of accuracy (also referred to as "position accuracy degree") of the position of a meta data target part highlighted on the endoscopic image Ic, wherein the position accuracy degree is the matching degree by the matching unit 32 between the reconstructed data Mr and the preliminary examination model Mp. It is herein assumed that the matching degree increases with an increase in the degree of matching between the reconstructed data Mr and the preliminary examination model Mp. Furthermore, in the third display example, the display control unit 33 displays the expected arrival distance from the tip unit 38 of the endoscope 3 to the meta data target part.

In the example shown in FIG. 8, the display control unit 33 displays the mark 41B circling the meta data target part on the endoscopic image Ic and displays the meta data display field 42C representing the content of the meta data relating to the meta data target part. Here, the display control unit 33 displays, in the meta data display field 42C, the diagnostic result (a comments "possible lesion part" herein) obtained in the preliminary examination.

Furthermore, the display control unit 33 displays a predicted arrival distance display field 45 representing the expected arrival distance from the tip unit 38 to the displayed meta data target point. For example, since parameters indicating the shooting position are obtained in the SfM or the like used for generating the reconstructed data Mr, the display control unit 33 calculates the expected arrival distance that is the distance between the position indicated by the parameters and the meta data target part based on the parameters.

Furthermore, the display control unit 33 displays a position accuracy degree display field 46 which displays a numerical value (65 in the value range from 0 to 100) representing the position accuracy degree and the gauge corresponding to the numerical value on the examiner confirmation screen image. In this case, for example, the display control unit 33 acquires, as the matching degree, the value, corresponding to the solution (optimum solution), of the evaluation function (e.g., the sum of the distance between each feature point of the preliminary examination model Mp and the corresponding feature point of the reconstructed data Mr) used in the matching by the matching unit 32. Then, the display control unit 33 calculates the position accuracy degree that is a value obtained by normalizing the matching degree to range from 0 to 100 by any normalization method. If the matching is performed to minimize the evaluation function, the display control unit 33 increases the position accuracy degree with a decrease in the value of the evaluation function corresponding to the solution. If the matching is performed to maximize the evaluation function, the display control unit 33 increases the position accuracy degree with an increase in the value of the evaluation function corresponding to the solution.

As described above, the display control unit 33 displays the position accuracy degree of the position of the meta data target part on the endoscopic image Ic and the endoscopic image Ic in which the meta data target part is highlighted. Then, according to the third display example, the display control unit 33 displays the position accuracy degree together with the endoscopic image Ic in which the meta data target part is highlighted. Thereby it is possible for the examiner to recognize the reliability of the position of the highlighted meta data target part. Further, the display control unit 33 displays the expected arrival distance that is useful information for the operation of the endoscope 3, thereby suitably supporting the operation of the examiner of the endoscopic examination. Any processing block in the image processing device 1 other than the display control unit 33 may calculate the position accuracy degree on the basis of the matching result by the matching unit 32.

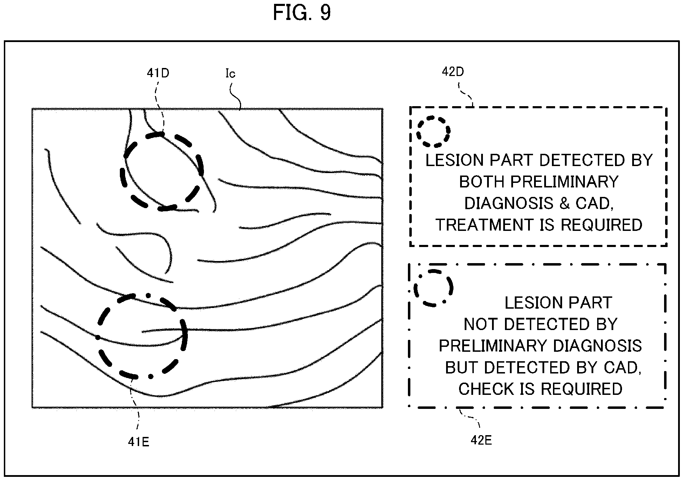

FIG. 9 shows a fourth display example of the examiner confirmation screen image. In the fourth display example, the display control unit 33 detects the lesion part in the endoscopic image Ic by executing a CAD in real time, and displays the information based on the comparison result between the position of the lesion part based on the CAD and the meta data target part.

In FIG. 9, the display control unit 33 displays the endoscopic image Ic on which a mark 41D and a mark 41E are superimposed, wherein the mark 41D circles the overlapping position between the meta data target part (i.e., the position of the lesion part detected in the preliminary examination) and the position of the lesion part detected by the CAD using the endoscopic image Ic and the mark 41E circles the position detected as the lesion part by the CAD instead of the meta data target part. Furthermore, the display control unit 33 displays the comment display field 42D in which a comment corresponding to the position highlighted by the mark 41D is displayed, and the comment display field 42E in which a comment corresponding to the position highlighted by the mark 41E is displayed.

Here, the display control unit 33 displays, in the comment display field 42D corresponding to the mark 41D, such information that the position indicated by the mark 41D in both the diagnostics in the preliminary examination and the CAD in real time is detected as a lesion part. Further, the display control unit 33 displays, in the comment display field 42E corresponding to the mark 41E, such information that the lesion part which was not detected in the preliminary examination has been detected by the CAD in real time. Even upon determining that there is a part which was detected in the preliminary examination as a lesion part but has not been detected by the CAD in real time, the display control unit 33 may provide the mark and the comment display field regarding the part to thereby inform the examiner of the endoscopic examination of the existence of the part.

Thus, in the fourth display example, the display control unit 33 notifies the examiner of the endoscopic examination of the information based on the comparison result between the diagnosis in the preliminary examination and the diagnosis by the CAD in real time. Thus, the display control unit 33 can suitably assist the examiner to conduct the endoscopic examination.

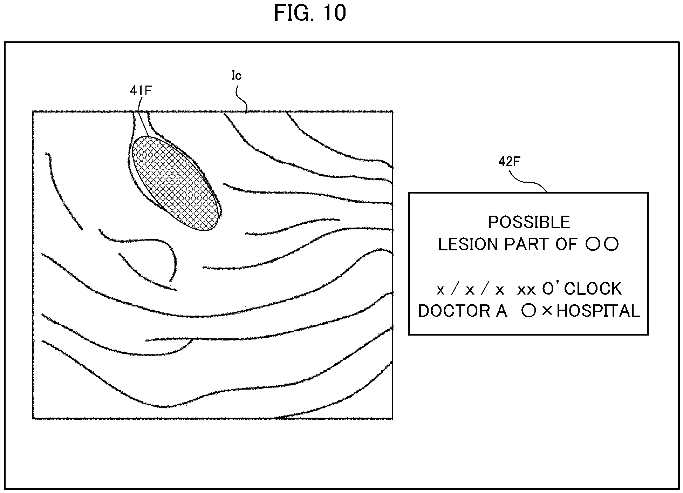

FIG. 10 shows a fifth display example of the examiner confirmation screen image. In the fifth display example, the display control unit 33 performs a process of extracting the lesion region including the meta data target part from the endoscopic image Ic and emphasizes and displays the extracted lesion region on the endoscopic image Ic.

In FIG. 10, the display control unit 33 highlights and displays on the endoscopic image Ic the lesion region 41F in the endoscopic image Ic including the meta data target part while displaying the meta data display field 42F indicating the content of the meta data relating to the lesion region 41F.

In the fifth display example, if there is a meta data target part in the target endoscopic image Ic of display, the display control unit 33 performs a process of extracting the lesion region 41F in the endoscopic image Ic including the meta data target part. In this instance, the display control unit 33 extracts the above-described lesion region 41F on the basis of information outputted by a lesion region extraction model by inputting the target endoscopic image Ic of display and a meta data target part into the lesion region extraction model, for example. In this case, the lesion region extraction model is trained to output, when the endoscopic image Ic and position information indicating the lesion part in the endoscopic image Ic are inputted thereto, information indicating the entire area of the lesion region including the position indicated by the position information, wherein the learned parameters are stored in advance in the memory 12 or the like. If a region has already been specified by the meta data as the meta data target part, the display control unit 33 may display the lesion region 41F on the basis of the meta data.

Thus, according to the fifth display example, the lesion region can be accurately extracted based on the meta data generated by the preliminary examination, and the lesion region can be displayed on the examiner confirmation screen image together with the diagnosis result obtained in the preliminary examination.

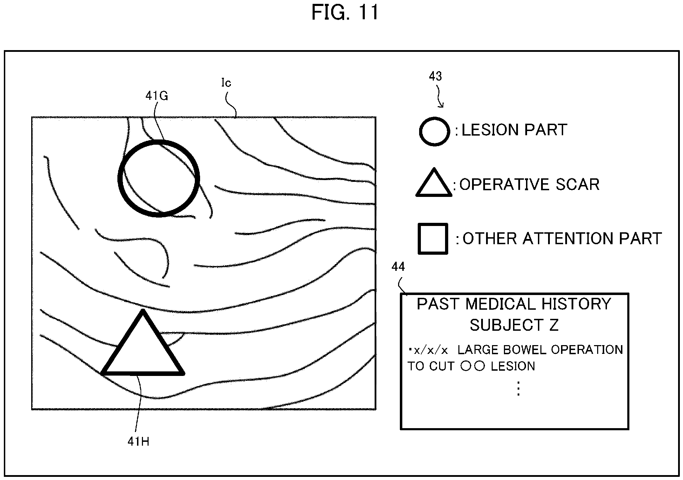

FIG. 11 shows a sixth display example of the examiner confirmation screen image. In the sixth display example, when the meta data includes the information on any attention part other than the lesion part, the display control unit 33 determines the display mode of the meta data target part according to the type of the attention part indicated by the meta data.

The display control unit 33 displays, on the examiner confirmation screen image, an endoscopic image Ic on which the marks 41G and 41H displayed according to the respective types of the attention parts is superimposed, a mark explanation display field 43 indicating the correspondence between the type of each mark to be possibly used and the corresponding type of the attention part, and a history display field 44 in which the past medical history of the subject is displayed. In the example shown in FIG. 10, the display control unit 33 recognizes, on the basis of the meta data, that there are a lesion part and an operative scar in the target endoscopic image Ic of display, and displays the endoscopic image Ic on which a circular mark 41G and a triangular mark 41H are superimposed, wherein the mark 41G circles the meta data target part corresponding to the lesion part and the mark 41H circles the meta data target part corresponding to the operative scar. Thus, the display control unit 33 allows the examiner to easily grasp the types of the attention parts detected in the preliminary examination.

The display control unit 33 provides a historical display field 44 indicating the past medical history of the subject (here "subject Z"). In this case, for example, the display control unit 33 receives information regarding the past medical history of the subject from the memory 12 or an external device which stores the past medical history, and displays the past medical history display field 44 based on the received information. Such information is preferably referred to by the examiner as reference information in endoscopic examination.

### (6) Modifications

In the case of detecting a lesion part based on the endoscopic image Ic according to the fourth display example or the like, the image processing device 1 may detect the lesion part using both the preliminary scan data and the endoscopic image Ic.

In this case, the image processing device 1 uses a lesion detection model configured to receive an input of an endoscopic image Ic and image (e.g., a CT image) of preliminary scanned data corresponding to the endoscopic image Ic (i.e., indicating the same photographed region as the endoscopic image Ic). In this case, the lesion detection model is trained to output, when receiving an input of the endoscopic image Ic and the image of the preliminary scan data, information indicating the presence or absence of a lesion part in the endoscopic image Ic and the position information regarding the lesion part in the case of the presence of the lesion part. In this instance, the endoscopic image Ic and the image of the preliminary scan data may be inputted to the lesion detection model in a state where they are stacked along the channel direction, or an integrated image obtained by arranging these images in the vertical or horizontal direction may be inputted to the lesion detection model. It is noted that the image processing device 1 identifies the image of the preliminary scan data corresponding to the endoscopic image Ic, for example, based on the matching result Rm obtained from the matching unit 32. The image processing device 1 inputs the endoscopic image Ic and the image of the preliminary scan data to the lesion detection model, and acquires, from the lesion detection model, information indicating the presence or absence of a lesion part in the endoscopic image Ic and the position information regarding the lesion part when the lesion part is present.

According to this modification, the image processing device 1 can detect the lesion part with higher accuracy using both the preliminary scan data and the endoscopic image Ic.

### <Second Example Embodiment>

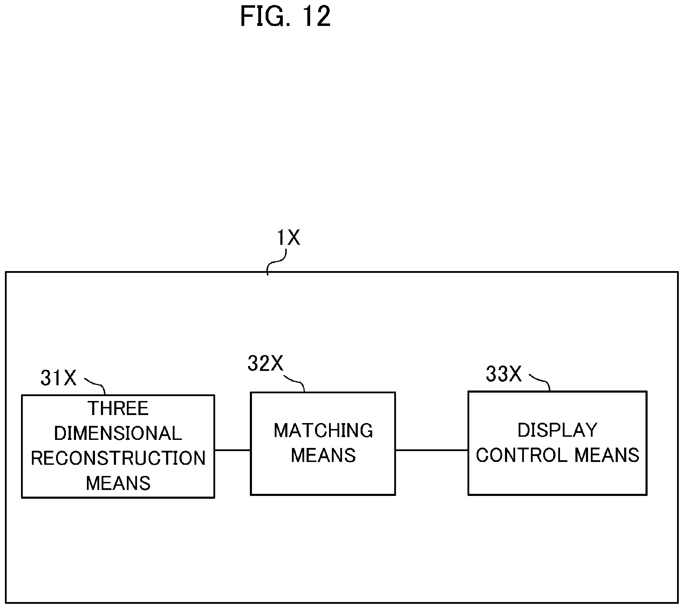

FIG. 12 is a block diagram of an image processing device IX according to the second example embodiment. The image processing device 1X mainly includes a three-dimensional reconstruction means 31X, a matching means 32X, and a display control means 33X. The image processing device 1X may be configured by a plurality of devices.

The three-dimensional reconstruction means 31X is configured to generate reconstructed data obtained by three-dimensionally reconstructing an examination target, based on endoscopic images of the examination target captured by a photographing unit provided in an endoscope. In this instance, the three-dimensional reconstruction means 31X may receive the endoscopic images directly from the photographing unit, or may acquire the endoscopic images from a storage device that stores the endoscopic images captured by the photographing unit. Further, the term "examination target" may be a large bowel, or may be any other organ such as a stomach. Examples of the three-dimensional reconstruction means 31X include the three-dimensional reconstruction unit 31 in the first example embodiment.

The matching means 32X is configured to perform matching between a three-dimensional model of the examination target and the reconstructed data. Here, the matching means 32X may acquire the three-dimensional model of the examination target from the memory of the image processing device 1X or from an external device other than the image processing device 1X. Examples of the matching means 32X include the matching unit 32 according to the first example embodiment.

The display control means 33X is configured to display information regarding meta data associated with the three-dimensional model, based on a result of the matching. The display control means 33X may display the information described above on a display unit of the image processing device 1X or may display the information described above on a display device separate from the image processing device 1X. Examples of the display control means 33X include a display control unit 33 in the first example embodiment.

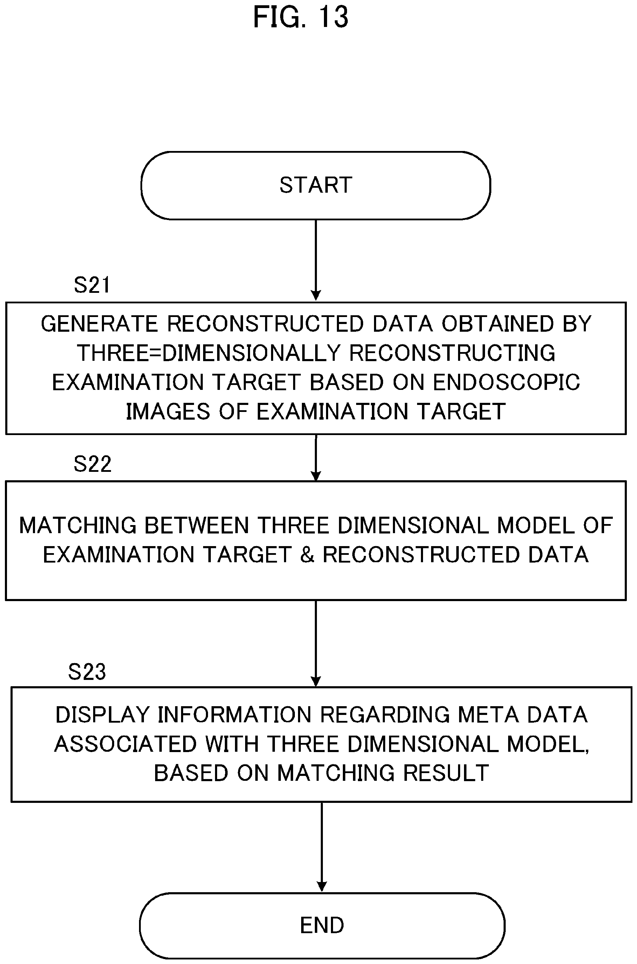

FIG. 13 is an exemplary flowchart illustrating a processing sequence that is executed by the image processing device 1X in the second example embodiment. The three-dimensional reconstruction means 31X generates reconstructed data obtained by three-dimensionally reconstructing an examination target, based on endoscopic images of the examination target captured by a photographing unit provided in an endoscope (step S21). The matching means 32X performs matching between a three-dimensional model of the examination target and the reconstructed data (step S22). The display control means 33X displays information regarding meta data associated with the three-dimensional model, based on a result of the matching (step S23).

According to the second example embodiment, the image processing device 1X can display information regarding the meta data preliminarily associated with the three-dimensional model of the examination target.

The whole or a part of the example embodiments described above (including modifications, the same applies hereinafter) can be described as, but not limited to, the following Supplementary Notes.

### [Supplementary Note 1]

An image processing device comprising:
a three-dimensional reconstruction means configured to generate reconstructed data obtained by three-dimensionally reconstructing an examination target, based on endoscopic images of the examination target captured by a photographing unit provided in an endoscope;
a matching means configured to perform matching between a three-dimensional model of the examination target and the reconstructed data; and
a display control means configured to display information regarding meta data associated with the three-dimensional model, based on a result of the matching.

### [Supplementary Note 2]

The image processing device according to Supplementary Note 1,
wherein the meta data includes position information representing a meta data target part that is a target of the meta data, and
wherein, upon determining that the meta data target part is included in an endoscopic image based on the result of the matching and the position information, the display control means is configured to display the endoscopic image in which the meta data target part is highlighted.

### [Supplementary Note 3]

The image processing device according to Supplementary Note 2,
wherein examples of the meta data target part include a part diagnosed as a lesion part.

### [Supplementary Note 4]

The image processing device according to Supplementary Note 2 or 3,
wherein the meta data includes information indicating a diagnosis result regarding the meta data target part, and
wherein the display control means is configured to display the information indicating the diagnosis result in association with the displayed endoscopic image in which the meta data target part is highlighted.

### [Supplementary Note 5]

The image processing device according to Supplementary Note 4,
wherein the meta data includes information indicating the diagnosis result and an attribute of one or more persons who made the diagnosis for the meta data target, and
wherein the display control means is configured to display information indicating the diagnosis result and the attribute in association with the displayed endoscopic image in which the meta data target part is highlighted.

### [Supplementary Note 6]

The image processing device according to Supplementary Note 5,
wherein in a case that the one or more persons are plural, the display control means is configured to differentiate a display mode of the information regarding the meta data for each of one or more persons from each other.

### [Supplementary Note 7]

The image processing device according to any one of Supplementary Notes 2 to 6,
wherein the display control means is configured to display a degree of position accuracy of the meta data target part on the endoscopic image and the endoscopic image in which the meta data target part is highlighted, and
wherein the degree of position accuracy is calculated based on the result of the matching.

### [Supplementary Note 8]

The image processing device according to any one of Supplementary Notes 2 to 7,
wherein the meta data target part is a part diagnosed as a lesion part, and
wherein the display control means is configured to display information based on a comparison result between a detected position of a lesion part based on the endoscopic image and the meta data target part.

### [Supplementary Note 9]

The image processing device according to any one of Supplementary Notes 2 to 8,
wherein the display control means is configured to determine a display mode relating to the meta data target part based on the type of the meta data target part.

### [Supplementary Note 10]

The image processing device according to any one of Supplementary Notes 2 to 9,
wherein, if the meta data target part is a part diagnosed as a lesion part, the display control means is configured to
extract a lesion region including the meta data target part and
highlight the lesion region in the displayed endoscopic image.

### [Supplementary Note 11]

The image processing device according to any one of Supplementary Notes 1 to 10,
wherein the three-dimensional model is data generated based on scan data of the examination target obtained in a preliminary examination conducted prior to the examination by the endoscope, and
wherein the meta data is data generated based on the examination result of the preliminary examination.

### [Supplementary Note 12]

An image processing method executed by a computer, the image processing method comprising:
generating reconstructed data obtained by three-dimensionally reconstructing an examination target, based on endoscopic images of the examination target captured by a photographing unit provided in an endoscope;
performing matching between a three-dimensional model of the examination target and the reconstructed data; and
displaying information regarding meta data associated with the three-dimensional model, based on a result of the matching.

### [Supplementary Note 13]

A storage medium storing a program executed by a computer, the program causing the computer to:
generate reconstructed data obtained by three-dimensionally reconstructing an examination target, based on endoscopic images of the examination target captured by a photographing unit provided in an endoscope;
perform matching between a three-dimensional model of the examination target and the reconstructed data; and
display information regarding meta data associated with the three-dimensional model, based on a result of the matching.

While the invention has been particularly shown and described with reference to example embodiments thereof, the invention is not limited to these example embodiments. It will be understood by those of ordinary skilled in the art that various changes in form and details may be made therein without departing from the spirit and scope of the present invention as defined by the claims. In other words, it is needless to say that the present invention includes various modifications that could be made by a person skilled in the art according to the entire disclosure including the scope of the claims, and the technical philosophy. All Patent and Non-Patent Literatures mentioned in this specification are incorporated by reference in its entirety.

### DESCRIPTION OF REFERENCE NUMERALS

- 1, 1X: Image processing device
- 2: Display device
- 3: Endoscope
- 11: Processor
- 12: Memory
- 13: Interface
- 14: Input unit
- 15: Light source unit
- 16: Audio output unit
- 100: Endoscopic examination system

## Claims

1. An image processing device comprising:
a three-dimensional reconstruction means configured to generate reconstructed data obtained by three-dimensionally reconstructing an examination target, based on endoscopic images of the examination target captured by a photographing unit provided in an endoscope;
a matching means configured to perform matching between a three-dimensional model of the examination target and the reconstructed data; and
a display control means configured to display information regarding meta data associated with the three-dimensional model, based on a result of the matching.

2. The image processing device according to claim 1,
wherein the meta data includes position information representing a meta data target part that is a target of the meta data, and
wherein, upon determining that the meta data target part is included in an endoscopic image based on the result of the matching and the position information, the display control means is configured to display the endoscopic image in which the meta data target part is highlighted.

3. The image processing device according to claim 2,
wherein examples of the meta data target part include a part diagnosed as a lesion part.

4. The image processing device according to claim 2 or 3,
wherein the meta data includes information indicating a diagnosis result regarding the meta data target part, and
wherein the display control means is configured to display the information indicating the diagnosis result in association with the displayed endoscopic image in which the meta data target part is highlighted.

5. The image processing device according to claim 4,
wherein the meta data includes information indicating the diagnosis result and an attribute of one or more persons who made the diagnosis for the meta data target, and
wherein the display control means is configured to display information indicating the diagnosis result and the attribute in association with the displayed endoscopic image in which the meta data target part is highlighted.

6. The image processing device according to claim 5,
wherein in a case that the one or more persons are plural, the display control means is configured to differentiate a display mode of the information regarding the meta data for each of one or more persons from each other.

7. The image processing device according to any one of claims 2 to 6,
wherein the display control means is configured to display a degree of position accuracy of the meta data target part on the endoscopic image and the endoscopic image in which the meta data target part is highlighted, and
wherein the degree of position accuracy is calculated based on the result of the matching.

8. The image processing device according to any one of claims 2 to 7,
wherein the meta data target part is a part diagnosed as a lesion part, and
wherein the display control means is configured to display information based on a comparison result between a detected position of a lesion part based on the endoscopic image and the meta data target part.

9. The image processing device according to any one of claims 2 to 8,
wherein the display control means is configured to determine a display mode relating to the meta data target part based on the type of the meta data target part.

10. The image processing device according to any one of claims 2 to 9,
wherein, if the meta data target part is a part diagnosed as a lesion part, the display control means is configured to
extract a lesion region including the meta data target part and
highlight the lesion region in the displayed endoscopic image.

11. The image processing device according to any one of claims 1 to 10,
wherein the three-dimensional model is data generated based on scan data of the examination target obtained in a preliminary examination conducted prior to the examination by the endoscope, and
wherein the meta data is data generated based on the examination result of the preliminary examination.

12. An image processing method executed by a computer, the image processing method comprising:
generating reconstructed data obtained by three-dimensionally reconstructing an examination target, based on endoscopic images of the examination target captured by a photographing unit provided in an endoscope;
performing matching between a three-dimensional model of the examination target and the reconstructed data; and
displaying information regarding meta data associated with the three-dimensional model, based on a result of the matching.

13. A storage medium storing a program executed by a computer, the program causing the computer to:
generate reconstructed data obtained by three-dimensionally reconstructing an examination target, based on endoscopic images of the examination target captured by a photographing unit provided in an endoscope;
perform matching between a three-dimensional model of the examination target and the reconstructed data; and
display information regarding meta data associated with the three-dimensional model, based on a result of the matching.
